# EUROPÄISCHE PATENTSCHRIFT

(11) **EP 1 515 934 B1**
(45) Veröffentlichungstag und Bekanntmachung des Hinweises auf die Patenterteilung: **08.10.2008**
(21) Anmeldenummer: 03712086.2
(22) Anmeldetag: 25.03.2003
(51) Int. Cl.: C07C 29/16, C07C 45/50, C07C 29/141, C08K 5/12, C11D 7/26

(54) **VERFAHREN ZUR HERSTELLUNG VON C13-ALKOHOLGEMISCHEN**
METHOD FOR PRODUCING C13-ALCOHOL MIXTURES
PROCEDE DE FABRICATION DE MELANGES DE C13-ALCOOLS

(30) Priorität: 10.05.2002 DE 10220799
(43) Veröffentlichungstag der Anmeldung: 23.03.2005
(73) Patentinhaber: Evonik Oxeno GmbH, 45772 Marl (DE)
(72) Erfinder: KAIZIK, Alfred, 45772 Marl (DE); TÖTSCH, Walter, 45770 Marl (DE); DROSTE, Wilhelm, 45770 Marl (DE); BÜSCHKEN, Wilfried, 45721 Haltern am See (DE); RÖTTGER, Dirk, 45657 Recklinghausen (DE); WIESE, Klaus-Diether, 45721 Haltern am See (DE)
(74) Vertreter: Hirsch, Hans-Ludwig
(86) Internationale Anmeldenummer: PCT/EP2003/003066
(87) Internationale Veröffentlichungsnummer: WO 2003/095402

(56) Entgegenhaltungen:
- EP-A- 0 402 051
- EP-A- 0 987 241
- WO-A-01/96508
- DE-A- 19 955 593

## Beschreibung

Die vorliegende Erfindung betrifft ein Verfahren zur Herstellung eines C₁₃-Alkoholgemisches, das insbesondere als Vorstufe für die Herstellung von Verbindungen mit tensidischen Eigenschaften und von Weichmachern geeignet ist.

Alkohole mit etwa 8 bis 20 Kohlenstoffatome sind Vorstufen für die Herstellung von nichtionischen und anionischen Tensiden. Für die Herstellung von Tensiden werden die Alkohole, funktionalisiert, wie beispielsweise alkoxyliert oder glycosidiert. Die beispielsweise erhaltenen Alkoxylate können entweder direkt als nichtionische oberflächenaktive Substanzen eingesetzt oder durch weitere Funktionalisierung, beispielsweise durch Sulfatierung oder Phosphatierung, in anionische oberflächenaktive Substanzen übergeführt werden. Die anwendungstechnischen Eigenschaften dieser Tenside, wie deren Netzvermögen, Schaumbildung, Fettlösevermögen, biologische Abbaubarkeit usw., werden durch die Kettenlänge und den Verzweigungsgrad des hydrophoben Kohlenwasserstoffrestes des eingesetzten Alkohols mitbestimmt. Alkohole, die für die Herstellung von Tensiden geeignet sind, werden oft als Tensidalkohole bezeichnet.

Tensidalkohole können durch Hydrierung von reinen Fettsäuren, von Gemischen von Fettsäuren, Estern von Fettsäuren und deren Gemischen gewonnen werden. Diese sogenannten Fettalkohole sind linear und haben im Wesentlichen eine gerade Anzahl von Kohlenstoffatomen und 12 bis 18 Kohlenstoffatome. Nachteilig für die Erzeugung von Tensiden auf Basis von Fettalkoholen ist, dass diese meistens nicht als reiner Stoff, sondern nur als Gemisch von Fettalkoholen mit unterschiedlicher C-Zahl zur Verfügung stehen, ihr hoher Preis, und dass sie nicht immer in ausreichender Menge vorhanden sind.

Tensidalkohole können weiterhin aus Ethylen nach dem Alfolprozess gewonnen werden, wobei als Koppelprodukt ein Aluminiumsalz entsteht.

Ein weiterer Weg zur Herstellung von Tensidalkoholen besteht darin, geeignete Olefine zu hydroformylieren und die dabei entstandenen Aldehyde zu hydrieren. Die dafür eingesetzten Olefine oder Olefingemische bestimmen u. a. die Linearität der Alkohole und können auf unterschiedliche Weise erzeugt werden.

Aus Paraffinen können durch Chlorierung und anschließender Dehydrochlorierung Olefine hergestellt werden, die zur Herstellung von Tensidalkoholen eingesetzt werden können. Diese Olefine haben den Nachteil, dass sie Chlorverbindungen enthalten, die die weitere Synthese stören könnten und dass keine Chlor-freien Endprodukte garantiert werden können.

Olefine zur Herstellung von Tensidalkoholen können ebenfalls durch Oligomerisierung von Ethylen gewonnen werden. Ein Verfahren zur Herstellung von Olefinen mit bestimmten Kettenlängen aus Ethylen ist der SHOP-Prozess (Shell Higher Olefine Process). Nachteilig an den auf Ethylen basierenden Verfahren zur Gewinnung der zur Herstellung von Tensidalkoholen geeigneten Olefine ist der hohe Preis des Ethylens.

Olefine, die zur Herstellung von Tensidalkoholen geeignet sind, können auch durch Oligomerisierung von C₃ bis C₆-Olefinen, wie insbesondere Propen oder Buten oder Gemische davon, erhalten werden.

Hierfür gibt es verschiedene technisch ausgeübte Verfahren.
Die Oligomerisierung von niedrigeren Olefinen an sauren Katalysatoren, wie Phosphorsäure auf Trägem oder sauren Zeolithe, liefert hochverzweigte Oligomerisate. Die Oligomerisierung an speziellen Nickelkatalysatoren ergibt weniger verzweigte Produkte. Beim Dimersol-Prozess (siehe Revue de 1 Institut Francais du Petrole, Vol. 37, Nr. 5, Sept./ Okt. 1982, S.639 ff) werden C₃- oder C₄-Olefine mit Hilfe eines im Reaktionsgemisch homogen gelösten Nickelhaltigen Katalysator oligomerisiert. Typische Katalysatorsysteme sind Nickel (0)-Komplexe in Kombination mit Lewis-Säuren wie AlCl₃, BF₃, SbF₅ usw. oder Ni(II)-Komplexe in Verbindung mit Alkylaluminiumhalogeniden. Ein Nachteil dieser homogenkatalytischen Verfahren besteht in der aufwendigen Abtrennung des Katalysators vom Reaktionsgemisch. Ein weiterer Nachteil ist, dass die erhaltenen höheren Olefine meistens mit Halogenspuren verunreinigt sind, die vom Oligomerisationskatalysator herrühren und mit diesen nicht vollständig entfernt werden. Diese Halogenspuren können die folgenden Reaktionsschritte beeinträchtigen und es werden keine Halogen-freie Endprodukte erhalten.
Die Oligomerisierung an speziellen heterogenen Nickelträgerkatalysatoren, wie beispielsweise im Octol-Prozess der Oxeno GmbH, liefert geringer verzweigte Produkte wie das Dimersol-Verfahren und hat den Vorteil, dass Katalysator- und Halogen-freie Produkte entstehen (siehe: J. Schulze, M. Homann, C4-Hydrocarbons and Derivatives, Springer-Verlag 1989, Seite 71).

Die Herstellung von C₁₃-Alkoholen durch Hydroformylierung von C₁₂-Olefinen mit nachfolgender Hydrierung der so erhaltenen Aldehyde ist bekannt.

DE 199 55 593 offenbart ein Verfahren zur Herstellung von C₁₃-Aloholgemischen, bei dem C₁₂-Olefine in Gegenwart eines Kobaltkatalysators hydroformyliert und die so erhaltenen Aldehyde hydriert werden. Eine Ausbeute ist nicht offenlegt. In DE 199 39 491 A1 (Beispiel 2) wird die Herstellung eines C₁₃-Alkoholgemisches durch Hydroformylierung eines Butentrimergemisches in Gegenwart eines Kobaltkatalysators und durch anschließende Hydrierung des Hydroformylats beschrieben. Die Ausbeute an C₁₃-Alkoholgemisch beträgt bezogen auf das C₁₂-Olefingemisch nur 80,4 %.

EP-A-0 987 241 beschreibt ein Verfahren zur selektiven Hydrierung von Monoolefinen, z. B. von Tri-n-Buten. Die Hydroformylierung mit Rhodium- oder vorzugsweise Kobaltkatalysatoren vorgenommen werden, die modifiziert oder unmodifiziert sein können.

In EP-A-0 402 051 wird die Hydroformylierung von Dodecen in Gegenwart von Kobaltkatalysatoren durchgeführt.

In WO 01/96508A, welches die Hydroformylierung eines durch Metathese erhaltenen Ausgangsproduktes beschreibt, werden als geeignete Katalysatoren sowohl Kobalt- als auch Rhodium-Katalysatoren in modifizierter oder unmodifizierter Form als geeignete Katalysatoren angegeben.

Hydroformylierungen mit Rhodium werden, abgesehen von kurzkettigen Olefinen, wie Propen und Butene, großtechnisch nur für C₈-Olefinisomerengemisch (Dibuten) durchgeführt, siehe Cornils et al., "Applied Homogeneous Catalysis with Organometallic Compounds", Vol. 1, VCH Verlag Weinheim, 1996, S. 35, S61 ff.. Eine detaillierte Beschreibung der Hydroformylierung von C₈-Olefinen ist in DE 33 38 340 und EP 0 272 608 offengelegt.

Längerkettige, verzweigte Olefine werden vorzugsweise nicht mit Rhodium sondern Kobaltkatalysiert hydroformyliert, was auf die mangelnde Aktivität und Selektivität des Rhodium-Katalysators zurückzuführen ist (Cornils et al, S. 64).

Bevorzugte Produkte von Hydroformylierungsreaktionen sind in der Regel die linearen, endständigen Aldehyde. Dies ist insbesondere dann von Bedeutung, wenn das Folgeprodukt der Aldehydhydrierung, der Alkohol als Tensid eingesetzt wird. Da bei C₁₂-Olefinen rein statistisch die Doppelbindung häufig innenständig ist bzw. durch den Herstellungsprozess mittels Butentrimerisierung meist zusätzlich verzweigte C₁₂-Olefine gebildet werden, ist für die Herstellung von C₁₃-Alkoholen ein Hydroformylierungsverfahren zweckmäßig, bei dem der Verzweigungsgrad der erhaltenen Aldehyde bzw. Alkohole nicht noch weiter erhöht wird. Dies würde gemäß Falbe et al. "New Syntheses with Carbon Monoxide", Springer-Verlag Berlin, 1980, S. 38 ff, S. 99, durch den Einsatz von unmodifizierten Rhodium statt Kobalt als Katalysator eintreten. Folgerichtig wird in DE 199 55 593 ein unmodifizierter Kobaltkatalysator eingesetzt.

Überraschender Weise wurde festgestellt, dass die Hydroformylierung von C₁₂-Olefinen mit modifizierten Rhodiumkatalysatoren durchführbar ist; so wurden sogar höhere Selektivitäten und Ausbeuten mit vergleichbaren Linearitäten wie beim entsprechend kobaltkatalysierten Prozess erhalten.

Die erfindungsgemäß hergestellten Alkohole (ggf. nach Hydrierung der Aldehyde) sind für die Herstellung von Tensiden und Weichmachern gut geeignet.

Gegenstand der Erfindung ist demnach ein Verfahren zur Herstellung eines C₁₃-Alkoholgemisch,
a) Oligomerisierung eines Butene enthaltenen Kohlenwasserstoffgemisch an einem Nickelträgerkatalysator,
b) Abtrennung der C₁₂-Olefinfraktion aus dem Reaktionsgemisch,
c) Hydroformylierung der C₁₂-Olefine
d) Abtrennung des Katalysators
e) Hydrierung des entkatalysierten Hydroformylierungsgemisches zum C₁₃-Alkoholgemisch,
dadurch gekennzeichnet,
dass die Hydroformylierung der C₁₂-Olefinfraktion c) in Gegenwart eines Rhodiumphosphit-, Rhodiumphosphonit- oder Rhodiumphosphinit-Katalysators vorgenommen wird.

Unmodifizierte Rhodiumkatalysatoren weisen als Liganden nur Kohlenmonoxid (vorhanden durch den Hydroformylierungsprozess) auf. Die Verwendung dieser Katalysatoren ist nicht Gegenstand der Erfindung, sondern ausschließlich der Einsatz modifizierter Rhodiumkatalysatoren, welche die koordinierenden Liganden Phosphite, Phosphonite, oder Phosphinite aufweisen.

### Einsatzstoffe

Im erfindungsgemäßen Verfahren werden in Schritt a) bevorzugt C₄-Ströme eingesetzt, die keine Olefine mit anderen C-Zahlen und weitestgehend keine mehrfach ungesättigte Kohlenwasserstoffe enthalten. Dabei kann der Gehalt an Isobuten, bezogen auf die Gesamtheit der Butene, bevorzugt unter 5 Massen-%, insbesondere unter 3 Massen-%, ganz besonders unter 1 Massen-% liegen.

Verwendbare technische Gemische sind Leichtbenzinfraktionen aus Raffinerien, C₄-Fraktionen aus FC- oder Steamcrackern, Gemische aus Fischer-Tropsch-Synthesen, Gemische aus Dehydrierung von Butanen, Gemische, entstanden durch Olefinmetathese oder anderen technischen Prozessen.

Aus den obigen Produktgemischungen können C₄-Gemische, die als Olefine im Wesentlichen lineare Butene enthalten, abgetrennt werden. Beispielsweise können für das erfindungsgemäße Verfahren geeignete Gemische linearer Butene aus der C₄-Fraktion eines Steamcrackers gewonnen werden. Dabei wird im ersten Schritt Butadien entfernt. Dies geschieht entweder durch Extraktion bzw. extraktive Destillation des Butadiens oder dessen Selektivhydrierung. In beiden Fällen wird ein praktisch butadienfreier C₄-Schnitt erhalten, das Raffinat I. Im zweiten Schritt wird Isobuten aus dem C₄-Strom entfernt, z. B. durch dessen Umsetzung mit Methanol zu Methyl-tert.-butylether (MTBE). Andere Möglichkeiten sind die Umsetzung des Isobutens aus dem Raffinat I mit Wasser zu tert.-Butanol oder die sauer katalysierte Oligomerisierung des Isobutens zu Diisobuten. Der jetzt Isobuten-freie C₄-Schnitt, das Raffinat II, enthält, wie gewünscht, die linearen Butene und gegebenenfalls Butane. Optional kann noch das 1-Buten destillativ abgetrennt werden. Beide Fraktionen, die mit But-1-en oder die mit But-2-enen, können im erfindungsgemäßen Verfahren eingesetzt werden.

Eine weitere Möglichkeit, ein geeignetes Edukt herzustellen, besteht darin, Raffinat I oder ein ähnlich zusammengesetztes Kohlenwasserstoffgemisch zu hydroisomerisieren. Dabei kann u. a. ein Gemisch, das aus 2-Butenen und gegebenenfalls n-Butan besteht, gewonnen werden. Ein entsprechendes Verfahren wird beispielsweise in DE 101 52 842.6 beschrieben.

In den Einsatzströmen für die Oligomerisierung werden gegebenenfalls noch vorhandene Mengen an mehrfach ungesättigten Kohlenwasserstoffen (Butadien) durch Selektivhydrierung auf Konzentrationen unter 10 Massen-ppm, insbesondere 5 Massen-ppm, ganz besonders 1 Massen-ppm gebracht. Es ist zweckmäßig, gegebenenfalls vorhandene Spuren an Sauerstoffhaltigen Verbindungen, wie Alkohole, Aldehyde, Ketone oder Ether, und/oder Schwefelhaltige Verbindungen zu entfernen. Dazu kann der C₄-Kohlenwasserstoffstrom über ein Absorptionsmittel, wie z. B. ein Molekularsieb, insbesondere eines mit einem Porendurchmesser von > 0,4 bis 0,5 nm, geleitet werden. Die Konzentration an Sauerstoffhaltigen Verbindungen im C₄-Kohlenwasserstoffstrom beträgt vorzugsweise weniger als 1 Massen-ppm, insbesondere weniger als 0,5 Massen-ppm.

### a) Oligomerisierung

Die Oligomerisierung der Butene erfolgt bevorzugt an Nickelträgerkatalysatoren. Als Trägermaterialien können beispielsweise Siliciumdioxid, Aluminiumoxid, Alumosilicate, Zeolithe, Zirkoniumoxid oder Titandioxid, gegebenenfalls nach Sulfatierung, eingesetzt werden. Besonders bevorzugt werden Titan-freie Nickelträgerkatalysatoren eingesetzt.

Für die Herstellung der Katalysatoren gibt es verschiedene Wege. Beispielweise können Katalysatoren durch gemeinsames Fällen von Nickelverbindungen und Trägermaterial, wie z. B. Aluminium- und/oder Siliciumverbindungen, Filtrieren und Tempern hergestellt werden. Eine weitere Möglichkeit besteht darin, Nickelverbindungen auf eines der oben genannten Trägermaterialien aufzubringen, beispielsweise durch Imprägnieren oder Aufsprühen, und anschließend den Katalysatorvorläufer zu calcinieren. Zur Herstellung der Katalysatoren können Nickelverbindungen, wie beispielsweise Nickelnitrat, Nickelsulfat, Nickelchlorid oder deren Amminkomplexe, eingesetzt werden.

Im erfindungsgemäßen Verfahren werden bevorzugt Katalysatoren eingesetzt, die formal aus Nickeloxid, Aluminiumoxid und Siliciumoxid bestehen. Diese Katalysatoren enthalten 5 bis 50 Massen-% Nickel, insbesondere 10 bis 30 Massen-%. Die Gehalte an Aluminium liegen im Bereich von 5 bis 30 Massen-%, insbesondere im Bereich von 7 bis 20 Massen-%. Die Massenanteile an Silicium liegen zwischen 10 und 40 %, insbesondere zwischen 20 und 30 %. Als weitere Kompononenten können diese Katalysatoren 0,1 bis 2 Massen-% Alkalioxid, Erdalkalioxid, Lanthanoxid oder Oxide der Seltenen Erden und gegebenenfalls Formgegungshilfsmittel enthalten.

Die Katalysatoren werden zweckmäßig in einer Form eingesetzt, in der sie einen geringen Stömungswiderstand bieten, z. B. von Granalien, Pellets oder Formkörpern, wie Tabletten, Zylinder, Kugeln, Strangextrudaten oder Ringen.

Die Oligomerisierung kann chargenweise oder kontinuierlich in allen Reaktoren, die üblicherweise bei Feststoff/Flüssigkeits-Kontaktreaktionen zum Einsatz gelangen, durchgeführt werden. Bei der Verwendung von kontinuierlich arbeitenden Strömungsreaktoren bedient man sich meistens, jedoch nicht ausschließlich, eines Festbetts. Wenn ein Festbett-Strömungsreaktor verwendet wird, kann die Flüssigkeit aufwärts oder abwärts strömen. Meistens wird ein Abwärtsströmen der Flüssigkeit bevorzugt. Weiterhin ist es möglich, den Reaktor unter Produktrückführung oder im geraden Durchgang zu betreiben.

Zur Erzielung eines hohen Butenumsatzes wird die Oligomerisierung in mehreren hintereinandergeschalteten Reaktoren durchgeführt, bevorzugt unter Produktrückführung. Dabei werden von den Reaktionsausträgen zunächst die Oligomeren abgetrennt und Teilmengen der verbleibenden Reste in die Zuläufe der entsprechenden Reaktoren rückgeführt.

Die Anzahl der in Reihe geschalteten Reaktoren liegt zwischen 1 und 10, bevorzugt zwischen 1 und 4.

Jeder Reaktor kann adiabatisch, polytrop oder praktisch isotherm, d. h. mit einem Temperaturanstieg unter 10°C, betrieben werden.

Die Temperaturen, bei denen die Reaktoren betrieben werden, liegen zwischen 20 und 200 °C, vorzugsweise zwischen 70 und 160 °C, ganz besonders zwischen 80 und 120 °C.

Die Umsetzung kann bei einem Druck gleich oder über dem Dampfdruck des EinsatzKohlenwasserstoffgemisches bei der jeweiligen Reaktionstemperatur durchgeführt werden, vorzugsweise bei einem Druck unter 40 bar. Um in den Reaktoren Verdampfungsprobleme zu vermeiden, sollte der Druck 2 bis 4 bar höher als der Dampfdruck des Reaktionsgemisches bei der höchsten Temperatur im Reaktor sein.

### b) Abtrennung der C₁₂-Olefine

Das Reaktionsgemisch der Oligomerisierung besteht aus nicht umgesetzten Butenen, gegebenenfalls aus n-Butan und Isobutan, und Oligomeren mit unterschiedlicher Molmasse (C₈-Olefinen, C₁₂-Olefinen, C₁₆-Olefinen, ...). Aus diesem Gemisch wird in einem oder mehreren Trennschritten die C₁₂-Olefinfraktion isoliert. Geeignete Trennvorrichtungen sind die üblichen, dem Fachmann bekannten Apparaturen. Dazu zählen beispielsweise Destillationskolonnen, wie Füllkörperkolonnen, Gewebepackungskolonnen oder Bodenkolonnen, die gewünschtenfalls mit Glocken, Siebplatten, Siebböden, Ventilen, seitenabzügen usw. ausgerüstet sein können, Verdampfer, wie Dünnschichtverdampfer, Fallfilmverdampfer, Wischblattverdampfer, Sambay-Verdampfer usw. und Kombinationen davon. Bevorzugt erfolgt die Abtrennung der C₁₂-Olefinfraktion durch fraktionierte Destillation.

### c) Hydroformylierung

Die abgetrennte C₁₂-Olefinfraktion wird zur Herstellung des erfindungsgemäßen C₁₃-Alkoholgemisches hydroformyliert und anschließend hydriert. Im erfindungsgemäßen Verfahren werden zur Erzielung einer hohen Ausbeute an C₁₃-Aldehyden und/oder den entsprechenden Alkoholen die Hydroformylierung in Gegenwart von modifizierten Rhodiumkatalysatoren durchgeführt.

Diese Rhodiumkatalysatoren können in Form ihrer aktiven Komplexe in den Prozess eingebracht werden, technisch ist es in der Regel aber einfacher, die aktiven Katalysatoren in situ aus stabilen, leicht lagerbaren Rhodiumverbindungen zu generieren. Geeignete Rhodiumverbindungen dafür sind zum Beispiel Rhodium(II)- und Rhodium(III)-salze, wie Rhodium(III)chlorid, Rhodium(III)nitrat, Rhodium(III)sulfat, Kalium-Rhodiumsulfat, Rhodium(II)- bzw. Rhodium(III)-carboxylat, Rhodium(II)- und Rhodium(III)acetat, Rhodium(II)octanoat, Rhodium(II)nonanoat, Rhodium(III)oxid, Salze der Rhodium(III)säure, Trisammoniumhexachlororhodat(III). Weiterhin eignen sich Rhodiumkomplexe, wie Rhodiumbiscarbonylacetylacetonat, Acetylacetonatobisethylenrhodium(I). Besonders geeignet sind Rhodiumacetat, Rhodiumoctanoat und Rhodiumnonanoat.

Im erfindungsgemäßen Verfahren wird ungefähr 1 bis 200 und vorzugsweise 1 bis 50, ganz besonders bevorzugt 1 bis 10 Mol Ligand pro Mol Rhodium zugesetzt. Frischer Ligand kann zu jedem Zeitprodukt der Reaktion zugesetzt werden, um die Konzentration an freiem Liganden konstant zu halten.

Im erfindungsgemäßen Verfahren beträgt die Konzentration des Rhodiums im Hydroformylierungsreaktor 1-100 Massen-ppm, insbesondere 1-50 Massen-ppm, ganz besonders 1-20 Massen-ppm, ganz besonders bevorzugt bei 1-10 Massen-ppm.

Im erfindungsgemäßen Verfahren werden Liganden eingesetzt, die mit Rhodium Komplexe bilden, die auch die Hydroformylierung von innenständigen und verzweigten Olefinen katalysieren.

Die Liganden sind organische Verbindungen, die Phosphoratome enthalten. Die Liganden können ein oder mehrzähnig sein, bei chiralen Liganden kann sowohl das Racemat als auch ein Enantiomer oder Diastereomer eingesetzt werden. Ebenfalls ist es möglich, ein Gemisch zweier oder mehrerer verschiedener Liganden zu verwenden. Als Phosphorliganden werden insbesondere diejenigen eingesetzt, die mit Rhodium weniger stabile Komplexe bilden als Triphenylphosphin, wie beispielsweise Phosphinoxide, Phosphite, Phosphonite und Phosphinite. Bevorzugt wird die Hydroformylierung der C₁₂-Olefinfraktion in Gegenwart von Rhodium-Phosphit-, Rhodium-Phosphinit- oder Rhodium-Phosphonit-Katalysatoren durchgeführt.

Beispiele für einsetzbare Phosphite sind Trimethylphosphit, Triethylphosphit, Tri-n-propylphosphit, Tri-i-propylphosphit, Tri-n-butylphosphit, Tri-i-butylphosphit, Tri-t-butylphosphit, Tris(2-ethylhexyl)phosphit, Triphenylphosphit, Tris(2-t-butyl-4-methoxyphenyl)phosphit, Tris(2-t-butyl-4-methylphenyl)phosphit, Tris(p-kresyl)phosphit. Außerdem sterisch gehinderte Phosphitliganden, wie sie unter anderem in EP 155. 508, US 4 668 651, US 4 748 261, US 4 769 498, US 4 774 361, US 4 835 299, US 4 885 401, US 5 059 710, US 5 113 022, US 5 179 055, US 5 260 491, US 5 264 616, US 5 288 918, US 5 360 938, EP 472 071, EP 518 241 und WO 97/20795 beschriebenen werden. Bevorzugt eingesetzt werden mit jeweils 1 oder 2 Isopropyl- und/oder tert.-Butylgruppen an den Phenylringen, vorzugsweise in ortho-Position zur Phosphitestergruppierung, substituierte Triphenylphosphite. Ein ganz besonders bevorzugter Ligand ist Tris(2.4-di-t-butylphenyl)phosphit.

Beispiele für einsetzbare Phosphonite sind Methyldiethoxyphosphin, Phenyldimethoxyphosphin, Phenyldiphenoxyphosphin, 6-Phenoxy-6H-dibenz[c,e][1,2]oxaphosphorin und dessen Derivate, in denen die Wasserstoffatome ganz oder teilweise durch Alkyl-, Arylreste oder Halogenatome ersetzt sind und Liganden die in den Patenten WO 9843935, JP 09-268152 und DE 198 10 794 und in den deutschen Patentanmeldungen DE 199 54 721 und DE 199 54 510 beschrieben werden.

Gängige Phosphinitliganden sind unter anderem in US 5 710 344, WO 95 06627, US 5 360 938, JP 07082281 beschrieben. Beispiele hierfür sind Diphenyl(phenoxy)phosphin und dessen Derivate, in denen die Wasserstoffatome ganz oder teilweise durch Alkyl-, Arylreste oder Halogenatome ersetzt sind, Diphenyl(methoxy)phosphin, Diphenyl(ethoxy)phosphin usw.

Im erfindungsgemäßen Verfahren wird die Rhodium-katalysierte Hydroformylierungen bei Drücken von 60 bis 300 bar durchgeführt, vorzugsweise bei Drücken von 150 bis 270 bar.

Die Temperaturen für die Rhodium-katalysierte Hydroformylierung liegen im Bereich von 40°C bis 180°C, bevorzugt bei 90°C bis 150°C, insbesondere bei 100 bis 130°C .

### d) Abtrennung des Katalysators

Nach der Hydroformylierung wird der größte Teil des Synthesegases durch Druckentspannung entfernt. Aus dem flüssigem Reaktionsaustrag werden Katalysator und Podukte destillativ getrennt. Der Katalysator und gegebenenfalls zugesetzte Liganden, Stabilisatoren usw. verbleiben im/als Destillationsrückstand. Beim Anfahren, oder wenn im Prozess nur wenig Hochsieder gebildet wird, kann es vorteilhaft sein, ein hochsiedendes (höher siedend als Produkte und Edukte), inertes Lösungsmittel einzusetzen, in dem sich der Katalysator löst. Der im hochsiedenden Lösungsmittel gelöste Katalysator kann dann direkt in die Reaktoren zurückgefahren werden. Besonders vorteilhaft ist es, als hochsiedendes Lösungsmittel die im Prozess gebildeten hochsiedenden Nebenprodukte einzusetzen. Andere geeignete Lösungsmittel sind hochsiedene Ester, wie 2,2,4-Trimethylpentandiol-1,3-monoisobutyrat, das als Texanol im Handel ist.

Für die technische Ausführung der destillativen Katalysatorabtrennung sind verschiedene Verfahrensweisen anwendbar. Bevorzugt ist die Abtrennung der Katalysatorlösung über Fallfilm-, Kurzstrecken- oder Dünnschichtverdampfer oder Kombinationen aus diesen Apparaten. Der Vorteil einer solchen Kombination kann zum Beispiel darin liegen, in einem ersten Schritt noch gelöstes Synthesegas sowie ein Teil der Produkte und der noch vorhandenen Ausgangsolefine (zum Beispiel in einem Fallfilmverdampfer) abzutrennen, um dann in einem zweiten Schritt (zum Beispiel in einem Dünnschichtverdampfer), die endgültige Abtrennung des Katalysators vorzunehmen.

Die Destillationsdrücke liegen dabei zwischen 5 mbar und 1 bar, vorzugsweise zwischen 10 mbar und 100 mbar.

Die Destillationstemperaturen betragen 40 bis 180 °C, insbesondere 80 bis 150 °C.

Optional kann das Sumpfprodukt zusätzlich mit Kohlenmonoxid stabilisiert werden, wie es in DE 100 48 301.1 beschrieben ist.

Ein Teil des Sumpfproduktes wird zur Konstanthaltung der Hochsiederkonzentration im Hydroformylierungsreaktor ausgeschleust. Der andere Teil des Sumpfproduktes wird in den Hydroformylierungsreaktor zurückgefahren. Mit dem Ausschleusestrom wird auch ein Teil des Katalysators (Rhodium und Ligand) aus dem Prozess entfernt. Diese Mengen und andere Fehlmengen an Rhodium und Ligand müssen zur Aufrechterhaltung der oben beschriebenen Katalysatorkonzentration im Hydroformylierungsreaktor nachdosiert werden. Optional können aus dem Ausschleusestrom weitere Produkte, beispielsweise durch Destillation, abgetrennt werden.

Aus dem Ausschleusestrom kann nach bekannten Verfahren das Rhodium zurückgewonnen werden.

Die Brüden, die bei der Aufkonzentrierung anfallen, können destillativ in Aldehyde und Alkohole, Kohlenwasserstoffe und sonstige Nebenprodukte getrennt werden. Aus der Kohlenwasserstofffiraktion können gegebenenfalls Olefine gewonnen werden, die in den Prozess zurückgeführt werden können.

### e) Hydrierung

Der vom Katalysator befreite Hydroformylierungsaustrag oder die nach Destilation erhaltene Aldehyd-haltige Fraktion wird in üblicher Weise in gasförmiger oder bevorzugt in flüssiger Phase mit einem Wasserstoff-haltigen Gas hydriert.

Man kann zur Hydrierung z. B. Nickel-, Kupfer/Nickel-, Kupfer/Chrom-, Kupfer/Chrom/Nickel-, Zink/Chrom-, Nickel/Molybdän-Katalysatoren verwenden. Die Katalysatoren können trägerfrei sein oder die hydrieraktiven Stoffe bzw. ihre Vorläufer können auf Träger, wie beispielsweise Siliciumdioxid oder Aluminiumdioxid, aufgebracht sein.

Bevorzugte Katalysatoren, an denen die Hydroformylierungsgemische hydriert werden, enthalten, jeweils 0,3 -15 Massen-% Kupfer und Nickel sowie als Aktivatoren 0,05 -3,5 Massen-% Chrom und vorteilhaft 0,01 - 1,6 Massen-% , vorzugsweise 0,02 - 1,2 Massen-% einer Alkalikomponente auf einem Trägermaterial, vorzugsweise Aluminiumoxid oder Siliciumoxid. Die Mengenangaben beziehen sich auf den noch nicht reduzierten Katalysator. Die Alkalikomponente ist optional.
Die Katalysatoren werden vorteilhaft in einer Form eingesetzt, in der sie einen geringen Strömungswiderstand bieten, z. B. in Form von Granalien, Pellets oder Formkörpers, wie Tabletten, Zylindern, Strangextrudaten oder Ringen. Sie werden zweckmäßig vor ihren Einsatz aktiviert, z. B. durch Erhitzen in einem Wasserstoff-haltigen Gasstrom.

Die Hydrierung, bevorzugt eine Flüssigphasenhydrierung, wird im Druckbereich von 5 -200 bar, insbesondere im Bereich von 5-100 bar, ganz besonders im Bereich 15 -50 bar durchgeführt. Eine Hydrierung in der Gasphase kann auch bei niedrigeren Drücken, mit entsprechend großen Gasvolumina durchgeführt werden. Werden mehrere Hydrierungsreaktoren eingesetzt, können die Gesamtdrücke in den einzelnen Reaktoren innerhalb der genannten Druckgrenzen gleich oder verschieden sein.

Die Reaktionstemperaturen liegen zwischen 120 und 220 °C, insbesondere zwischen 140 und 180 °C. Beispiele für solche Hydrierungen sind in DE 198 42 369 und DE 198 42 370 beschrieben.

Aus dem nach der Hydrierung erhaltenen Reaktionsgemisch kann nach üblichen, dem Fachmann bekannten Verfahren, insbesondere durch fraktionierte Destillation, das erfindungsgemäße C₁₃-Alkoholgemisch gewonnen werden.

Optional kann auch das C₁₃-Alkoholgemisch in mehrere Fraktionen aufgetrennt werden.

Die Erfindung betrifft weiterhin Verfahren zur Herstellung von Weichmachern und von tensidisch wirkenden Verbindungen.

Weichmacher werden aus dem C₁₃-Alkoholgemisch durch Umsetzung mit Carbonsäuren oder deren Anhydriden, insbesondere Dicarbonsäuren oder deren Anhydriden, zu den entsprechenden Estern hergestellt. Das Verfahren zur Herstellung von Weichmachern gestaltet sich derart, das zunächst Alkohol nach dem Hauptanspruch hergestellt und dieser Alkohol in einer weiteren Verfahrensstufe
f) mit Phthalsäure, Phthalsäureanhydrid, Adipinsäure, Cyclohexandicarbonsäure und/oder Cyclohexandicarbonsäureanhydrid zum entsprechenden Ester umgesetzt wird.

Verfahren zur Herstellung tensidisch wirksamer Derivate des C₁₃-Alkoholgemisches weisen die Schritte (a) bis (e) als in Anspruch 1 definiert und eine der folgenden Reaktionsschritte auf:
g) Alkoxylierung
h) Glycosidierung
i) Sulfatisierung)
j) Phosphatisierung
k) Alkoxylierung mit nachfolgender Sulfatierung
   sukzessives Durchführen der Verfahrensstufen g) und i)
l) Alkoxylierung mit nachfolgender Phosphatierung
   sukzessives Durchführen der Verfahrensstufen g) und j)

Die Umsetzung der erfindungsgemäß hergestellten Tensidalkohole mit Alkylenoxiden zu den entsprechenden Alkoxylaten sowie deren Sulfatierung und/oder Phosphatierung ist in Kosswig/Stache, "Die Tenside", Carl Hanser Verlag, München, Wien, 1993, Kapitel 2.2 und 2.3 beschrieben.

Bei der Alkoxylierung des C₁₃-Alkoholgemisches zu den entsprechenden Alkoxylaten werden Alkylenoxid oder Alkylenoxidderivate und deren Mischungen eingesetzt. Bevorzugt werden Alkylenoxide mit 1 bis 18, insbesondere 1 bis 10, ganz besonders 1 bis 6 Kohlenstoffatom(en) verwendet. Insbesondere seien Ethylenoxid, Propylenoxid, Butenoxid und Mischungen davon genannt.

Die Umsetzung des C₁₃-Alkoholgemisches mit dem/den Alkylenoxid(en) erfolgt nach üblichen, dem Fachmann bekannten Verfahren und in den dafür üblichen Apparaturen.

Dabei können aus selbst aus einem C₁₃-Alkohol und einem Alkylenoxid eine Vielzahl von Derivatgemischen hergestellt werden, die sich durch die mittlere Molmasse und Molmassenverteilung unterscheiden.
Die mittlere Molmasse der Alkoxylate ist durch das Molmengenverhältnis von C₁₃-Alkoholgemisch zu Alkylenoxid festgelegt. Bevorzugt werden alkoxylierte C₁₃-Alkoholgemische mit 1 bis 100, insbesondere 1 bis 50, ganz besonders 1 bis 10 Alkylenoxideinheiten hergestellt. Die Molmassenverteilung ist vom ausgeübten Verfahren abhängig.

Das C₁₃-Alkoholgemisch kann mit einem Alkylenoxid oder mit zwei oder mit mehreren verschiedenen Alkylenoxiden umgesetzt werden. Bei der Umsetzung des C₁₃-Alkoholgemisches mit zwei oder mehreren verschiedenen Alkylenoxide können, abgesehen von mittlerer Molmasse und Molmasseverteilung, unterschiedliche Produktgemische entstehen. Wird das C₁₃-Alkoholgemisch mit einem Gemisch der unterschiedlichen Alkylenoxide umgesetzt, so addieren sich zu Beginn der Umsetzung bevorzugt die reaktiveren Alkylenoxide an das C₁₃-Alkoholgemisch, anschließend erfolgt ein mehr oder minder statistischer Einbau der unterschiedlichen Alkylenoxidgruppen.
Werden dagegen die verschiedenen Alkylenoxide getrennt nacheinander eingesetzt, so entstehen Alkoxylate, die im Wesentlichen entsprechend der Zugabereihenfolge die Alkylenoxideinheiten in Form von Blöcken einpolymerisiert enthalten.

Die Umsetzung von C₁₃-Alkoholgemischen mit Alkylenoxid(en) kann durch Säuren, Basen oder amphotere Stoffe katalysiert werden. Beispielsweise können als basische Katalysatoren Alkalihydroxide, Alkalialkoholate oder Erdalkalihydroxide sowie als Lewissäuren Aluminiumtrichlorid und Bortrifluorid, eingesetzt werden.

Je nach Reaktivität des/der eingesetzten Alkylenoxid(s)/(e) und abhängig vom angewandten Verfahren liegen die Alkoxylierungstemperaturen im Bereich von 70 bis 260 °C, insbesondere im Bereich von 90 bis 230 °C.

Der Druck liegt vorzugsweise zwischen Normaldruck und 300 bar. Gegenfalls kann das eingesetzte Alkylenoxid mit einer inerten Flüssigkeit oder mit einem Inertgas verdünnt sein.

Diese alkoxylierten Derivatgemische des C₁₃-Alkoholgemisches sind nicht-ionische Tenside mit hoher Oberflächenaktivität. Diese Gemische können daher in vielen Anwendungsbereichen eingesetzt werden, wie beispielsweise als Dispergiermittel, Papierhilfsmittel, Korrosionsinhibitor sowie als Hilfsmittel für Dispersionen.

Glycosierte C₁₃-Alkoholgemische können durch ein-, zwei- oder mehrfache Umsetzung von C₁₃-Alkoholgemischen mit Mono-, Di- oder Polysacchariden nach üblichen dem Fachmann bekannten Verfahren gewonnen werden.

Dabei können zwei verschiedene Synthesewege angewendet werden. Bei dem einen wird das C₁₃-Alkoholgemisch mit einem Saccharid unter Wasserabspaltung direkt zum Zielprodukt umgesetzt. Als Katalysator dafür werden Säuren eingesetzt, wie beispielsweise Salzsäure oder Schwefelsäure. Dabei entstehen meistens Oligosaccharide mit statistischer Kettenlängenverteilung. Nach der anderen Syntheseroute wird das Saccharid zunächst derivatisiert und das Zwischenprodukt mit dem C₁₃-Alkoholgemisch zum Zielprodukt umgesetzt. Das Zwischenprodukt kann ein Acetal sein, das durch Umsetzung eines Saccharids, gegebenfalls in Form einer wässrigen Lösung, mit einem Alkohol mit 1 bis 8 C-Atomen gewonnen wird, oder ein O-Acetalchlorosaccharid, das bei der Umsetzung eines Saccharids mit Chlorwasserstoff entsteht. Beide Zwischenprodukte ergeben bei der Umsetzung mit C₁₃-Alkoholgemischen die Zielprodukte. Bei dem Weg über die Chlorverbindung ist es zweckmäßig, zur Bindung der frei werdenden Säure eine Base zuzusetzen.

Für die Glycosidierung werden vorzugsweise Monosaccharide eingesetzt, wie beispielsweise Hexosen oder Pentosen, insbesondere Glucose.
Das C₁₃-Alkoholgemisch kann mit einem Sacharid, zwei oder-mehreren Sacchariden umgesetzt werden. Bei der Umsetzung des C₁₃-Alkoholgemisches mit zwei oder mehreren verschiedenen Sacchariden können, abgesehen von mittlerer Molmasse und Molmasseverteilung, unterschiedliche Produktgemische entstehen. Wird das C₁₃-Alkoholgemisch mit einem Gemisch der unterschiedlichen Sacchariden umgesetzt, so addieren sich zu Beginn der Umsetzung bevorzugt die reaktiveren Saccharide an das C₁₃-Alkoholgemisch, anschließend erfolgt ein mehr oder minder statistischer Einbau der unterschiedlichen Saccharidgruppen. Werden dagegen die verschiedenen Saccharide getrennt nacheinander eingesetzt, so entstehen Glycosylate, die im Wesentlichen entsprechend der Zugabereihenfolge die Saccharideinheiten in Form von Blöcken einpolymerisiert enthalten.

Die bei der Glycosidierung angewandten Verfahren und Reaktonsbedingungen sind z.B. in Ullmann's Encyclopedia of Industrial Chemistry, 5. Aufl., Bd. A25 (1994), S. 792-793 und den dort zitierten Literatustellen beschrieben.

Die Sulfatierung des C₁₃-Alkoholgemisches oder dessen Alkoxylaten zu den entsprechenden Folgeprodukten (Alkylsulfaten oder Alkylethersulfaten) erfolgt durch Umsetzung mit Schwefelsäure oder Schwefelsäurederivaten .

In US 3 462 525, US 3 420 875 oder US 3 524 864 werden Verfahren zur Sulfatierung von Alkoholen beschrieben. Diese Verfahren können zur Sulfatierung von C₁₃-Alkoholgemische oder deren Oxylaten genutzt werden. Weitere geeignete Verfahren zur Sulfatierung sind auch in Ullmann's s Encyclopedia of Industrial Chemistry, 5. Auflage, Bd. A25 (1994), S. 779-783 und in Kirk-Othmer Encylopedia of Chemical Technology. 4.Ed., Vol. 23 (1997), S. 500-502 und den dort zitierten Literaturstellen beschrieben
Für die Sulfatierung des erfindungsgemäß hergestellten C₁₃-Alkoholgemisch wird bevorzugt konzentrierte Schwefelsäure eingesetzt. Die Konzentration der Schwefelsäure ist vorzugsweise 75- bis 100 %-ig (bezogen auf die Masse), insbesondere 85- bis 98%-ig.

Zur Erzielung hoher Ausbeuten wird bei der Veresterung des C13-Alkoholgemisches das Molmengenverhältnis von Alkohol zu Sulfatierungsmittel bevorzugt 1/1 bis 1/1,5, insbesondere 1/1 bis 1/1,2 eingestellt.
Die Reaktionsbedingungen der Sulfatierung liegen bevorzugt in einem Temperaturbereich von Umgebungstemperatur 20 °C bis 80 °C, insbesondere im Bereich von 40 bis 75 °C.
Als weitere geeignete Sulfatierungsmittel können z. B. Schwefeltrioxid, Lösungen von Schwefeltrioxid in Schwefelsäure (Oleum), Chlorsulfonsäure und Sulfurylchlorid eingesetzt werden.. Wird Schwefelsäuretrioxid als Sulfatierungsmittel eingesetzt, so kann die Sulfatierung in einem Fallfilmverdampfer, bevorzugt im Gegenstrom, durchgeführt werden.
Die anschließende Produktaufarbeitung der Sulfatierung, wie z.B. die Neutralisation und die Abtrennung der gegebenenfalls eingesetzten Lösemittel, erfolgt nach üblichen, dem Fachmann bekannten Verfahren.

Die Phosphatierung des C₁₃-Alkoholgemisches oder dessen Alkoxylaten zu den gewünschten Folgeprodukten (Alkylphosphaten oder Alkyletherphoshaten) erfolgt bevorzugt durch Veresterung mit Phosphorsäure oder Phosphorsäurederivaten .
Die Phosphatierung des erfindungsgemäßen C₁₃-Alkoholgemisches und dessen alkoxylierten Produkte erfolgt im Allgemeinen in analoger Weise zur Sulfatierung.
Geeignete Verfahren zur Phosphatisierung von Alkoholen sind die dem Fachmann bekannten, wie z. B. in Kirk-Othmer Encylopedia of Chemical Technology. 4.Ed., Vol. 23 (1997), S. 504-506, und in R.L.Rudnick et al. "Synthetic Lubricants and High-Performace Functional Fluids" Marcel Dekker New York, 1999, S.103 ff. beschrieben werden.
Als geeignete Phosphatierungsmittel werden z. B. Phosphorsäure, Polyphosphorsäure, Phosphorpentoxid und Phosphorylchlorid eigesetzt.

Ein weiterer Gegenstand der Erfindung ist die Verwendung der erfindungsgemäß hergestellten funktionalisierten C₁₃-Alkoholgemische als Tenside, Dispergiermittel, Papierhilfsmittel, Korrosionsinhibitoren und als Hilfsmittel für Dispersionen.

Die folgenden Beispiele sollen die Erfindung erläutern, ohne ihre Anwendungsbreite einzuschränken, die sich aus der Beschreibung und den Patentansprüchen ergibt.

### Beispiel 1 Vergleichsbeispiel

### Hydroformylierung von C₁₂-Olefin (Tributen) in Gegenwart von Kobalt-Katalysator

In einem 21-Hochdruckautoklaven, der mit einem Rüher und elektrischer Beheizung versehen war, wurden 1000 g Tributen (C₁₂-Olefingemisch aus dem Octol-Prozess der OXENO GmbH) in Gegenwart von Kobaltkatalysator bei 180 °C und konstant gehaltenem Synthesegasdruck von 270 bar hydroformyliert. Das Synthesegas enthielt 50 Vol.-% CO und 50 Vol.-% H2.
Für die Herstellung des aktiven Kobalt-Katalysators wurde als Katalysator-Vorläufer eine wässrige Kobaltacetat-Lösung mit 1,35 Massen-% Co verwendet. Diese Kobaltacetat-Lösung wurde unter Rühren 5 h bei 170 °C und 280 bar mit Synthesegas behandelt. Nach Abkühlen auf Raumtemperatur und Entspannen wurden die gebildeten Kobaltcarbonyle durch Extraktion mit Edukt Tributen in die organische Phase überführt. Nach Abtrennen der wässrigen Phase wurde das mit Kobaltcarbonylen beladene Tributen mit einem Gehalt von 0,09 Massen-% Kobalt (gerechnet als Metall) unter den o. g. Reaktionsbedingungen 5 Stunden lang hydroformyliert. Der Olefin-Umsatz wurde sowohl mittels einer GC-Analyse als auch über die Menge an aufgenommenem Synthesegas verfolgt.

Nach Abkühlung auf Raumtemperatur wurde das Reaktionsgemisch entspannt aus dem Autoklaven entleert und durch Behandlung mit 5%-iger Essigsäure und Luft bei 80 °C vom Co-Katalysator befreit.

Nach GC-Analyse enthielt der Reaktionsaustrag 22,5 Massen-% C₁₂-Olefin, 23,5 Massen-% C₁₃-Aldehyd (Iso-Tridecanal), 44,3 Massen-% C₁₃-Alkohol (Iso-Tridecanol) und 9,7 Massen-% Hochsieder. Diese Produktzusammensetztung entspricht einem Tributen-Umsatz von 75,4 % und einer Wertproduktausbeute (C₁₃-Aldehyd/Alkohol) von 62,4 %.

### Beispiel 2 gemäß der Erfindung

### Hydroformylierung von C₁₂-Olefin (Tributen) in Gegenwart von Rhodium-Katalysator

In einem 21 Autoklav wurden 1000 g Tributen aus dem Octol-Prozess bei 135 °C unter 270 bar Synthesegasdruck 5 Stunden lang in Gegenwart eines Phosphit-modifizierten Rhodiumkatalysators umgesetzt. Der aktive Rhodiumkatalysator wurde in situ aus Rhodiumoctanoat und Tris(2.4-di-tert.butylphenyl)phosphit generiert.
Die Rhodiumkonzentration (bezogen auf Gesamtreaktionmasse) wurde auf 10 ppm eingestellt, das molare Phosphor -Rhodium-Verhältnis (P/Rh) betrug 10 zu 1.

Der Umsatz des Olefins wurde sowohl mittels einer GC-Analyse als auch über die Menge an aufgenommenem Synthesegas verfolgt. Nach 5 Stunden wurde die Reaktion abgebrochen. Der Reaktionsaustrag enthielt 13,1 Massen-% C₁₂-Olefin, 80,9 Massen-% C₁₃-Aldehyd (Iso-Tridecanal), 4,6 Massen-% C₁₃-Alkohol (Iso-Tridecanol) und 1,4 Massen-% Hochsieder. Diese Produktzusammensetztung entspricht einem Tributen-Umsatz von 85,0 % und einer Wertproduktausbeute (C₁₃-Aldehyd/Alkohol) von 83,0 %.

Gegenüber der Hydroformylierung von C₁₂-Olefinisomerngemisch in Gegenwart von Kobalt-Katalysator,wie im Beispiel 1 dargestellt, führt die Rhodium-katalysierte Hydroformylierung mit 83,0 % Wertproduktausbeute zur deutlichen Steigerung der Wertproduktausbeuten.

## Patentansprüche

1. Verfahren zur Herstellung eines C₁₃-Alkoholgemisches durch
a) Oligomerisation eines Butene enthaltenen Kohlenwasserstoffgemisch an einem Nickelträgerkatalysator,
b) Abtrennung der C₁₂-Olefinfraktion aus dem Reaktionsgemisch,
c) Hydroformylierung der C₁₂-Olefine
d) Abtrennung des Katalysators
e) Hydrierung des entkatalysierten Hydroformylierungsgemisches zum C₁₃-Alkoholgemisch,
**dadurch gekennzeichnet,**
**dass** die Hydroformylierung der C₁₂-Olefinfraktion c) in Gegenwart eines Rhodiumhosphit-, Rhodiumphosphonit- oder Rhodiumphosphinit-Katalysators vorgenommen wird.

2. Verfahren nach Anspruch 1,
**dadurch gekennzeichnet,**
**dass** die Hydroformylierung der C₁₂-Olefinfraktion c) in Gegenwart eines Katalysators, bestehend aus Rhodium und Tris (2.4-Di-t.-butylphenyl)phosphit, durchgeführt wird.

3. Verfahren nach Anspruch 1 oder 2,
**dadurch gekennzeichnet,**
**dass** die Oligomerisierung des Buten enthaltenden Kohlenwasserstoffgemisches an einen Nickelträgerkatalysator durchgeführt wird.

4. Verfahren nach Anspruch 3,
**dadurch gekennzeichnet,**
**dass** die Oligomerisierung des Buten enthaltenden Kohlenwasserstoffgemisches an einen Titan-freien Nickelträgerkatalysator durchgeführt wird.

5. Verfahren zur Herstellung von Weichmachern aus einem gemäß Anspruch 1 bis 4 hergestellten C₁₃-Alkoholgemisch durch 1) Veresterung des C₁₃-Alkoholgemischs mit Phthalsäure, Phthalsäureanhydrid, Adipinsäure, Cyclohexandicarbonsäure und/oder Cyclohexandicarbonsäureanhydrid.

6. Verfahren zur Alkoxylierung von C₁₃-Alkoholgemischen durch die Schritte a) bis e) gemäß Ansprüche 1-4 und
g) Umsetzung der C₁₃-Alkoholgemische mit Ethylenoxid, Propylenoxid und/oder Butylenoxid.

7. Verfahren zur Glycosidierung von C₁₃-Alkoholgemischen durch die Schritte a) bis e) gemäß Ansprüche 1-4 und
h) Säurekatalyiserte Umsetzung des C₁₃-Alkoholgemischs mit Mono-, Di-, und/oder Polysaccariden.

8. Verfahren zur Sulfatisierung von C₁₃-Alkoholgemischen durch die Schritte a) bis e) gemäß Ansprüche 1-4 und
i) Umsetzen des C₁₃-Alkoholgemisches mit Schwefelsäure, SO₃, Chlorsulfonsäure und/oder Sulfurylchlorid.

9. Verfahren zur Phosphatisierung von C₁₃-Alkoholgemischen durch die Schritte a) bis e) gemäß Ansprüche 1-4 und
j) Verestert der C₁₃-Alkoholgemische mit Phosphorsäure oder Phosphorsäurederivaten.

10. Verfahren zur Alkoxydierung mit nachfolgender Sulfatierung von C₁₃-Alkoholgemischen durch die Schritte a) bis e) gemäß Ansprüche 1-4 und
k) sukzessives Durchführender Verfahrensschritte g) und i) gemäß Ansprüche 6 und 8.

11. Verfahren zur Alkoxydierung mit nachfolgender Phosphatierung von C₁₃-Alkoholgemischen durch die Schritte a) bis e) gemäß Ansprüche 1-4 und durch
l) sukzessives Durchführen der Verfahrensschritte g) und j) gemäß Ansprüche 6 und 9.

## Claims

1. Process for preparing a C₁₃-alcohol mixture by
a) oligomerization of a butene-containing hydrocarbon mixture over a supported nickel catalyst,
b) separation of the C₁₂-olefin fraction from the reaction mixture,
c) hydroformylation of the C₁₂-olefins,
d) separation of the catalyst from the hydroformylation mixture and
e) hydrogenation of the hydroformylation mixture which has been freed of catalyst to give the C₁₃-alcohol mixture,
**characterized in that** the hydroformylation of the C₁₂-olefin fraction c) is carried out in the presence of a rhodium phosphite, rhodium phosphonite or rhodium phosphinite catalyst.

2. Process according to Claim 2, **characterized in that** the hydroformylation of the C₁₂-olefin fraction c) is carried out in the presence of a catalyst comprising rhodium and tris(2,4-di-t-butylphenyl) phosphite.

3. Process according to Claim 1 or 2, **characterized in that** the oligomerization of the butene-containing hydrocarbon mixture is carried out over a supported nickel catalyst.

4. Process according to Claim 3, **characterized in that** the oligomerization of the butene-containing hydrocarbon mixture is carried out over a titanium-free supported nickel catalyst.

5. Process for preparing plasticizers from a C₁₃-alcohol mixture prepared according to Claims 1 to 4 by
l) esterification of the C₁₃-alcohol mixture with phthalic acid, phthalic anhydride, adipic acid, cyclohexanedicarboxylic acid and/or cyclohexanedicarboxylic anhydride.

6. Process for the alkoxylation of C₁₃-alcohol mixtures by steps a) to e) according to Claims 1-4 and
g) reaction of the C₁₃-alcohol mixtures with ethylene oxide, propylene oxide and/or butylene oxide.

7. A process for the glycosidation of C₁₃-alcohol mixtures by steps a) to e) according to Claims 1-4 and
h) acid-catalyzed reaction of the C₁₃-alcohol mixture with monosaccharides, disaccharides and/or polysaccharides.

8. Process for the sulfation of C₁₃-alcohol mixtures by steps a) to e) according to Claims 1-4 and
i) reaction of the C₁₃-alcohol mixture with sulfuric acid, SO₃ chlorosulfonic acid and/or sulfuryl chloride.

9. Process for the phosphation of C₁₃-alcohol mixtures by steps a) to e) according to Claims 1-4 and
j) esterification of the C₁₃-alcohol mixtures with phosphoric acid or phosphoric acid derivatives.

10. Process for the alkoxylation and subsequent sulfation of C₁₃-alcohol mixtures by steps a) to e) according to Claims 1-4 and
k) carrying out process steps g) and i) according to Claims 6 and 8 in succession.

11. Process for the alkoxylation and subsequent phosphation of C₁₃-alcohol mixtures by steps a) to e) according to Claims 1-4 and
l) carrying out process steps g) and j) according to Claims 6 and 9 in succession.

## Revendications

1. Procédé pour la préparation d'un mélange d'alcools en C₁₃ , par
a) oligomérisation d'un mélange d'hydrocarbures contenant des butènes, sur un catalyseur au nickel sur support,
b) séparation de la fraction d'oléfines en C₁₂ d'avec le mélange réactionnel,
c) hydroformylation des oléfines en C₁₂,
d) séparation du catalyseur,
e) hydrogénation du mélange d'hydroformylation séparé du catalyseur, conduisant au mélange d'alcools en C₁₃,
**caractérisé en ce que** l'hydroformylation de la fraction d'oléfines en C₁₂ c) est effectuée en présence d'un catalyseur au phosphite-rhodium, phosphonite-rhodium ou phosphinite-rhodium.

2. Procédé selon la revendication 1, **caractérisé en ce que** l'hydroformylation de la fraction d'oléfines en C₁₂ c) est effectuée en présence d'un catalyseur constitué de rhodium et de phosphite de tris(2,4-di-tert-butylphényle).

3. Procédé selon la revendication 1 ou 2, **caractérisé en ce que** l'oligomérisation du mélange d'hydrocarbures contenant du butène est effectué sur un catalyseur au nickel sur support.

4. Procédé selon la revendication 3, **caractérisé en ce que** l'oligomérisation du mélange d'hydrocarbures contenant du butène est effectué sur un catalyseur au nickel sur support, exempt de titane.

5. Procédé pour la préparation de plastifiants à partir d'un mélange d'alcools en C₁₃ préparé selon l'une quelconque des revendications 1 à 4, par 1) estérification du mélange d'alcools en C₁₃ par l'acide phtalique, l'anhydride phtalique, l'acide adipique, l'acide cyclohexanedicarboxylique et/ou l'anhydride d'acide cyclohexanedicarboxylique.

6. Procédé pour l'alcoxylation de mélanges d'alcools en C₁₃ par les étapes a) à e) selon les revendications 1 à 4 et g) réaction des mélanges d'alcools en C₁₃ avec de l'oxyde d'éthylène, de l'oxyde de propylène et/ou de l'oxyde de butylène.

7. Procédé pour la glycosylation de mélanges d'alcools en C₁₃ par les étapes a) à e) selon les revendications 1 à 4 et h) réaction du mélange d'alcools en C₁₃ avec des mono-, di- et/ou polysaccharides, catalysée par un acide.

8. Procédé pour la sulfatation de mélanges d'alcools en C₁₃ par les étapes a) à e) selon les revendications 1 à 4 et i) réaction du mélange d'alcools en C₁₃ avec de l'acide sulfurique, SO₃, de l'acide chlorosulfonique et/ou du chlorure de sulfuryle.

9. Procédé pour la phosphatation de mélanges d'alcools en C₁₃ par les étapes a) à e) selon les revendications 1 à 4 et j) estérification des mélanges d'alcools en C₁₃ avec de l'acide phosphorique ou des dérivés d'acide phosphorique.

10. Procédé pour l'alcoxylation avec sulfatation subséquente de mélanges d'alcools en C₁₃ par les étapes a) à e) selon les revendications 1 à 4 et k) mise en oeuvre subséquente des étapes de processus g) et i) selon les revendications 6 et 8.

11. Procédé pour l'alcoxylation avec phosphatation subséquente de mélanges d'alcools en C₁₃ par les étapes a) à e) selon les revendications 1 à 4 et par 1) mise en oeuvre subséquente des étapes de processus g) et j) selon les revendications 6 et 9.
